# EUROPÄISCHE PATENTSCHRIFT

(11) **EP 0 838 046 B1**
(45) Veröffentlichungstag und Bekanntmachung des Hinweises auf die Patenterteilung: **13.09.2000**
(21) Anmeldenummer: 97922822.8
(22) Anmeldetag: 26.03.1997
(51) Int. Cl.: G04B 23/02, G04B 47/06

(54) **VERFAHREN UND VORRICHTUNG ZUM WECKEN VON SCHLAFENDEN VOR DER REM-PHASE EINES KURZEN ERHOLUNGSSCHLAFES**
METHOD AND DEVICE FOR WAKING PEOPLE SLEEPING BEFORE THE REM-PHASE OF A SHORT RECOVERY SLEEP
PROCEDE ET DISPOSITIF POUR REVEILLER DES PERSONNES ENDORMIES AVANT LA PHASE REM D'UN BREF SOMMEIL REGENERATEUR

(30) Priorität: 06.04.1996 DE 19614790
(43) Veröffentlichungstag der Anmeldung: 29.04.1998
(73) Patentinhaber: Güse, Klaus, 30171 Hannover (DE); Daghbouche, Belkacem, 30655 Hannover (DE); Zakaria, Edgar, 30455 Hannover (DE); Daghbouche, Karim, 30175 Hannover (DE)
(72) Erfinder: DAGHBOUCHE, Karim, D-30175 Hannover (DE)
(86) Internationale Anmeldenummer: DE9700640
(87) Internationale Veröffentlichungsnummer: WO9738359

(56) Entgegenhaltungen:
- US-A- 4 228 806
- PATENT ABSTRACTS OF JAPAN vol. 8, no. 117 (P-277), 31.Mai 1984 & JP 59 023284 A (KOGYO GIJUTSUIN), 6.Februar 1984,
- PATENT ABSTRACTS OF JAPAN vol. 13, no. 523 (C-657), 21.November 1989 & JP 01 212565 A (HIROYUKI KOMIYAMA), 25.August 1989,

## Beschreibung

### Anwendungsgebiet der Erfindung

Die Erfindung betrifft eine Vorrichtung und ein Verfahren zum Wecken von Schlafenden vor der REM-Phase eines kurzen Erholungsschlafes. Die Erfindung führt in Abhängigkeit eines bestimmten, individuell verschieden vorgegebenen Schlafquantums und feststellbaren Schlafzustandes zum Erwachen des Schlafenden.

Die Erfindung dient der Verbesserung der Lebensbedingungen durch Effizienzsteigerung von Schlaf, insbesondere des Mittagsschlafs.

### Charakteristik des bekannten Standes der Technik

Bekannt sind Verfahren zum selbsttätigen Wecken eines schlafenden Subjektes, bei denen die REM-Schlafphasen über Sensoren erfaßt und zum Auslösen des Weckvorganges herangezogen werden, um ein einfacheres und angenehmeres Aufwecken zu ermöglichen (vgl. JP 01 212565 A).

In DE 42 09 336 A1 wird darüberhinaus die Zykluszeit der REM-Schlafphasen ermittelt und die Weckzeit mit einer REM-Phase zur Deckung gebracht.

Zur Erfassung der physiologischen Daten, werden die Schlafphasen anhand der Augenbewegungen, der Hirnströme, der Herzschlagfrequenz, der Atemfrequenz, der Körper- bzw. Kopftemperatur, der Körperbewegung und/oder des Stoffwechselaufwandes erfaßt.

Bekannt sind Verfahren und Vorrichtungen, bei welchen zwecks angenehmen Aufwachens auf das Vorliegen des Schlafstadiums REM geschlossen werden soll, indem Körperbewegungen über einen unter einer Schlafmatratze fixierten Vibrationssensor erfaßt werden (vgl. JP 59 023284 A).

Bekannt sind Verfahren und Vorrichtungen zur Nutzung der unterschiedlichen Wach/Schlafphasen eines Menschen. Diese dienen der Beeinflussung von Einrichtungen, welche die jeweils aktuelle und/oder vorausberechenbare Wach/Schlafphase einer zu wecken den Person mit dem Weckverhalten eines gekoppelten Weckers koordinieren (vgl. EP 0 496 196 A1).

Auch hierbei werden körperbezogene Daten wie Körpertemperatur und/oder die Körperbewegungen des Schläfers und oder die Gehirnstromkurve (EEG) über die Schlafzeit gemessen.

Im Rahmen der standardisierten Verfahrenstechnik der Schlafphysiologie sind elektromyographische Verfahren und Vorrichtungen bekannt, bei welchen der *Muskeltonus* als physiologisches Signal mitherangezogen wird, damit ein schlafendes Subjekt ausschließlich in einer leichten Schlafphase (NREM 1 und 2) geweckt werden kann (vgl. US 4 228 806). Da der *Muskeltonus* bei Eintreten in eine Tiefschlafphase (NREM 3 und 4) leicht abfällt, wird in US 4 228 806 unter anderem vorgeschlagen, das Wecken eines Subjektes vom leichten Abfall des *Muskeltonus* abhängig zu machen, indem bei abfallendem *Muskeltonus* noch kein Weckreiz eingeleitet wird, und das Schlafintervall bis zum Wiederanstieg des *Muskeltonus* verlängert wird.

Nicht bekannt sind Vorrichtungen und Verfahren, die in Abhängigkeit des einem Wegfall entsprechenden Abfalls des *Muskeltonus* einer Extremität zu einem bestimmbaren Schlafzustand wecken.

Die standardisierte Verfahrenstechnik der Schlafphysiologie erfaßt den *Muskeltonus* über elektromyographische Verfahren (EMG). Hierbei wird nicht auf das Vorliegen eines Schlafzustandes REM geschlossen, obwohl der *Muskeltonus* gegenüber dem Wachsein und aller bekannten Schlafstadien Null wird. Dies ist darin begründet, daß beim Eintreten in das Stadium REM, der EMG-Kanal nicht erlischt, sondern das überlagerte Elektrokardiogramm (EKG) der Herzfrequenzmessung anzeigt (vgl. z.B. Koella, Werner P.; Die Physiologie des Schlafes: Eine Einführung; Fischer, Stuttgart, New York; 1988, S.28).

Erfindungsgemäß ist die Erfassung des *Muskeltonus* damit unabhängig von Körperbewegungen, da das Vorhandensein eines *Muskeltonus* nicht notwendig auch Körperbewegungen impliziert. Zudem sinkt der *Muskeltonus* bei Vorliegen einer REM-Schlafphase signifikant auf Null, womit keine Körperbewegungen außer gelegentliche Zuckungen mehr möglich sind. Darüber hinaus ist der Weckzeitpunkt zur REM-Phase ohne großen technischen Aufwand eindeutig bestimmbar, da außer vom *Muskeltonus* von allen anderen physiologischen Daten wie Herzfrequenz etc. aufgrund der gegenseitigen Überlagerungen abgesehen wird, und genau zum Zeitpunkt des erloschenen *Muskeltonus* geweckt wird.

### Ziel der Erfindung

Die Erfindung hat das Ziel, die Lebensbedingungen und die Leistungsfähigkeit des *homo sapiens* durch Gewährleistung eines physisch und psychisch günstigen Schlafablaufs, insbesondere eines günstigen Weckzeitpunktes, zu verbessern.

Die Erfindung hat das Ziel, die Konzentrations- und Leistungsfähigkeit des *homo sapiens* zu verbessern, indem in geringer Zeit eine hohe Revitalisierung ermöglicht wird, ohne daß ein physisch oder psychisch günstiger Schlafzustand unterbrochen werden müßte.

Die Erfindung soll dazu beitragen, günstige Arbeitszeitbedingungen besser zu nutzen, z.B. flexible Arbeitszeit, und ungünstige Arbeitszeitbedingungen möglichst zu mildern, z.B. lange Arbeits- und Bereitschaftsperioden oder Schichtarbeit.

Die Erfindung soll ferner dazu beitragen, durch Prävention von nichtvoluntativ eintretendem Schlaf, mittels autonom kontrolliert eingeleitetem Schlaf und gleichermaßen eingeleitetem Erwachen, Sicherheitsaspekten im privaten, öffentlichen und professionellen Gesellschaftsleben zu genügen.

### Darlegung des Wesens der Erfindung

Der Erfindung liegt die Aufgabe zugrunde, eine Vorrichtung und ein Verfahren zu kreieren, das Schlafende in geringer Zeit eine hohe Revitalisierung ermöglicht, ohne daß ein physisch oder psychisch günstiger Schlafzustand unterbrochen werden müßte. Diese Aufgabe wird mit einer Vorrichtung und mit Verfahrensschritten gelöst, wie sie in den Patentansprüchen 1 und 5 angegeben sind:

Demzufolge wird eine Extremität noch im Wachzustand aus einer entspannten Haltung in eine angespannte Haltung überführt, damit ein erhöhter *Muskeltonus* erzeugt wird.

Dieser *Muskeltonus* wird von einem Sensor registriert und versetzt den Schalter in Schaltzustand Null (0).

Der Schlafende wird durch das Eintreten in die REM-Phase eine signifikante Abnahme des *Muskeltonus* erleiden.

Der signifikant abgenommene *Muskeltonus* wird die Extremität in die entspannte Haltung zurückführen.

Dabei wird die entspannte Haltung der Extremität den Sensor instantan in Schaltzustand Eins (1) versetzen.

Der Schaltzustand Eins (1) führt zur Emittierung eines Weckreizes. Umgekehrt wird bei Schaltzustand Null (0) kein Weckreiz emittiert.

Die Schlafphase ist damit abhängig vom Schlafzustand "REM" oder "NREM", wobei die Schlafdauer auf die NREM-Phase beschränkt ist.

Die Erfindung ist ausgestaltbar, indem das Verfahren in andere Vorrichtungen integriert werden kann, welche über Sensoren mit zwei Schaltzuständen verfügen die in geeigneter Position zu einer Extremität, z.B. eines Fingers liegen, und welche über eine Weckreizeinheit verfügen, z.B. Mobilfunkgeräte (Handy).

Die Erfindung wird möglich, weil zur Bestimmung des Schlafzustandes (REM oder NREM) signifikante Unterschiede des *Muskeltonus* genutzt werden, da der *Muskeltonus* bei Vorliegen einer REM-Phase praktisch Null wird und sich damit signifikant von einer NREM-Phase unterscheidet, und weil die REM-Phase im Schlafverlauf des durchschnittlich gesunden *homo sapiens* , insbesondere beim Mittagsschlaf, relativ schnell einsetzt, und weil bis zum Einsetzen der ersten REM-Phase bereits hinreichend viele Schlafstadien zur physiologischen und psychologischen Revitalisierung durchlaufen sind.

Die Erfindung ist sinnvoll, weil es in jeder Gesellschaft, insbesondere in hochtechnisierten, Situationen gibt, in denen man zum Erhalt der Leistungs- und Konzentrationsfähigkeit bei eintretender Müdigkeit schlafen müßte, dieses aber aus persönlichen, gesellschaftlichen oder arbeitstechnischen Gründen nicht realisieren kann. Das führt zu einer Abnahme der Leistungs- und Konzentrationsfähigkeit, und damit zu einer verminderten Produktivität und zu einem Sicherheitsdefizit, insbesondere in professionellen Lebensbereichen.

Die Erfindung kommt dem Schlafbedürfnis in diesen Situationen insofern nach, als dem müden *homo sapiens* ein in Bezug auf die Müdigkeit ausreichend großes, als auch in Bezug auf die Situation hinreichend kleines Schlafquantum gewährt wird, wobei dieses durch geringen Aufwand an Technik, Gewicht und äußeren Dimensionen, sowie Gewährleistung hoher Benutzerfreundlichkeit und Betriebssicherheit realisiert wird.

### Ausführungsbeispiel

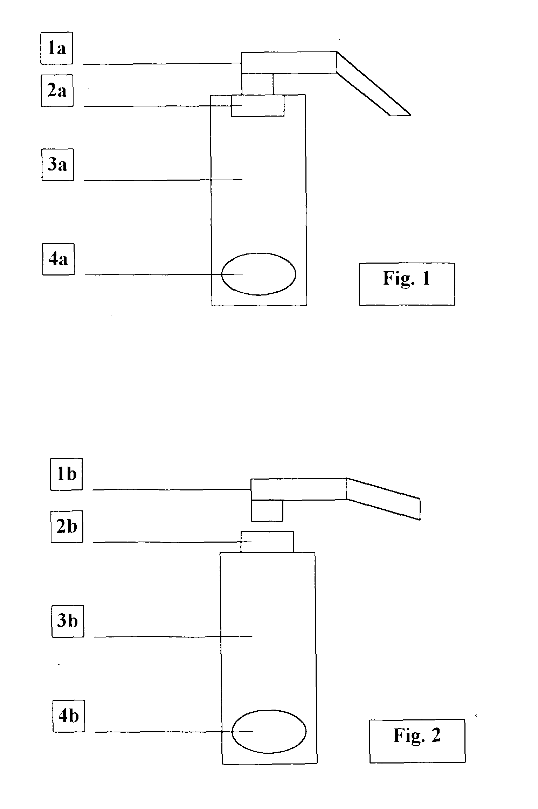

Die Erfindung soll nun an einem Ausführungsbeispiel näher erläutert werden. Die zugehörige Zeichnung zeigt in den Figuren 1 und 2 das Blockschema einer Vorrichtung zur Realisierung des Verfahrens.

Gemäß Fig. 1 und Fig. 2 besteht die Vorrichtung zur Realisierung des Verfahrens zum Wecken von Schlafenden vor der REM-Phase eines kurzen Erholungsschlafes, aus einem Sensor mit zwei Schaltzuständen (2a / 2b), einer Weckreizeinheit (4a / 4b), und einem Körper (3a / 3b) zur Integration des Sensors mit zwei Schaltzuständen und der Weckreizeinheit.

Aus Gründen der Übersichtlichkeit sind etwaige mechanische oder elektrische Energiequellen und etwaige mechanische oder elektrische Verbindungen/Schaltungen zwischen dem Sensor mit zwei Schaltzuständen und der Weckreizeinheit weggelassen worden.

Im ersten Schritt wird eine Extremität (1a) den Sensor mit zwei Schaltzuständen in eine bestimmte Schaltstellung bringen (2a), so daß die Weckreizeinheit keinen Weckreiz emittiert (4a). **(Fig. 1)** Solange sich der Schlafende in einer NREM-Phase befindet, wird der *Muskeltonus* die Extremität in dieser Position verharren lassen, so daß kein Weckreiz emittiert wird. **(Fig. 1)**

Im zweiten Schritt tritt der Schlafende in die REM-Phase ein, womit der *Muskeltonus* derart abfällt, daß die Extremität des Schlafenden in die entspannte Position übergeht (1b), und simultan dazu der Sensor mit zwei Schaltzuständen in eine Position übergeht (2b), die die Weckreizeinheit zum Emittieren eines Weckreizes veranlaßt (4b). **(Fig. 2)**

Der Weckreiz kann taktil, elektrisch, optisch, oder vorzugsweise durch ein akustisches Signal gegeben werden.

Die neue Vorrichtung und das neue Verfahren zum Wecken von Schlafenden vor der REM-Phase eines kurzen Erholungsschlafes läßt sich in beliebiger Körperhaltung und damit in beliebigen Situationen einsetzen, z.B. in einer Sitzhaltung.

## Patentansprüche

1. Verfahren zum Wecken von Schlafenden vor der ersten REM-Phase eines kurzen Erholungsschlafes, bei dem Sensorsignale zur Bestimmung des Weckzeitpunktes ausgewertet werden, wobei auf Grund dieser Auswertung der Weckreiz eingeleitet wird, **dadurch gekennzeichnet**, daß die genannte Auswertung den Abfall des *Muskeltonus* als Sensorsignal umfaßt.

2. Verfahren nach Anspruch 1, **dadurch gekennzeichnet**, daß die genannte, den Abfall des *Muskeltonus* als Sensorsignale umfassende Auswertung, jenen durch Bestimmung des *Muskeltonus* vom *Muskeltonus* einer Extremität ableitet.

3. Verfahren nach Anspruch 2, **dadurch gekennzeichnet**, daß der *Muskeltonus* von einem Finger abgeleitet wird.

4. Verfahren nach Anspruch 1, **dadurch gekennzeichnet**, daß der Weckreiz aus taktilen, elektrischen, optischen oder akustischen Reizen besteht.

5. Vorrichtung zur Durchführung des Verfahrens nach Anspruch 1 mit Mitteln zum Wecken von Schlafenden vor der ersten REM-Phase eines kurzen Erholungsschlafes die einen Sensor enthalten, dessen Signale einer Auswerteeinrichtung zur Bestimmung des Weckzeitpunktes zugeführt werden, sowie einer Weckreizeinheit zur Einleitung des Weckreizes aufgrund dieser Auswertung, **dadurch gekennzeichnet,** daß die genannte Auswerteeinrichtung einen Schalter umfaßt, der den Abfall des *Muskeltonus* als Sensorsignal empfängt, und über diesen Schalter die Weckreizeinheit betätigt.

6. Vorrichtung nach Anspruch 5, **dadurch gekennzeichnet,** daß ein mechanischer oder elektromechanischer Schalter die Weckreizeinheit betätigt.

7. Vorrichtung nach Anspruch 5, **dadurch gekennzeichnet**, daß ein elektrischer oder elektronischer Schalter die Weckreizeinheit betätigt.

8. Vorrichtung nach Anspruch 5, **dadurch gekennzeichnet**, daß ein photoelektrischer Sensor den *Muskeltonus* erfaßt.

## Claims

1. Method for waking people before the occurrence of REM-sleep within a short nap, whereby sensor signals are utilized to detect the appropriate time point of awakening, whereby due to this utilization the waking stimulation will occur, **wherein** the mentioned utilization contains the muscle tension's drop as sensor signal.

2. A method of claim 1, **wherein** the mentioned, the drop of the muscle tension as sensor signals containing utilization, is determined through muscle tension of the muscle tension of a body extremity.

3. Method of claim 2, **wherein** muscle tension is determined from a finger.

4. Method of claim 1, **wherein** the waking signal consists of a tactile, electric, optic, or acoustic signal.

5. Device to implement the method of claim 1, by means containing a sensor for a method for waking people before the occurrence of REM-sleep within a short nap, whose signals are transferred to a utilization unit as well to an alarm unit to initiate the waking stimulus, **wherein** the mentioned utilization unit contains a switch who receives the drop of the muscle tension as sensor signal and who triggers the alarm unit by this switch.

6. Device to claim 5, **wherein** the alarm unit is triggered by a mechanical or electromechanical switch.

7. Device to claim 5, **wherein** the alarm unit is triggered by an electrical or electronic switch.

8. Device to claim 5, **wherein** a photoelectric sensor is used to detect the muscle tension.

## Revendications

1. Procédure pour réveiller des dormeurs avant le sommeil paradoxal d'une courte sièste de récupération où on analyse des signeaux d'un capteur pour la définition du moment du réveil, à l'occasion de quoi ladite analyse instruit une sonnerie, **caractérisé en ce que** ladite analyse contient la baisse du *tonus musculaire* comme signal d'un capteur.

2. Procédure selon la revendication 1, **caractérisé en ce que** ladite analyse de la baisse du *tonus musculaire* en function d'un signal d'un capteur, se déduit par la définitition du *tonus musculaire* du *tonus musculaire* d'une extrémité (une de quarte membres plus le cou).

3. Procédure selon la revendication 2, **caractérisé en ce que** ledit *tonus musculaire* est déduit d'un doigt.

4. Procédure selon la revendication 1, **caractérisé en ce que** les stimulations de la sonnerie consitent en stimulations tactiles, électriques, optiques ou acoustiques.

5. Dispositif pour exécuter la procédure selon la revendication 1 avec des moyens pour réveiller des dormeurs avant le sommeil paradoxal d'une courte sièste de récupération qui contient un capteur, où ses signeaux sont transmit à un dispositif d'analyse de la définition du moment du réveil, ainsi qu'un dispositif de sonnerie pour l'instruction d'une stimulation de réveil par ladite analyse, **caractérisé en ce que** ledit dispositif d'analyse contient un interrupteur qui reçoit la baisse du *tonus musculaire* comme signal d'un capteur, et qui commande par ledit interrupteur le dispositif de sonnerie.

6. Dispositif selon la revendication 5, **caractérisé en ce qu'**un interrupteur mécanique ou électro-mécanique commande le dispositif de sonnerie.

7. Dispositif selon la revendication 5, **caractérisé en ce qu'**un interrupteur électrique ou électronique commande le dispositif de sonnerie.

8. Dispositif selon la revendication 5, **caractérisé en ce qu**'un capteur photo-électrique detect le *tonus musculaire.*
